Europäisches Patentamt

European Patent Office

Office européen des brevets

(11) Publication number: **0 550 883 A2**

## EUROPEAN PATENT APPLICATION

(21) Application number: **92121867.3**

(22) Date of filing: **23.12.92**

(51) Int. Cl.5: **C12Q 1/68**

(30) Priority: **09.01.92 US 819359**

(43) Date of publication of application:
**14.07.93 Bulletin 93/28**

(84) Designated Contracting States:
**AT BE CH DE DK ES FR GB GR IE IT LI LU NL PT SE**

(71) Applicant: **F. HOFFMANN-LA ROCHE AG**
**Grenzacherstrasse 124**
**CH-4002 Basel(CH)**

(72) Inventor: **Weiss, Judith Barbara**
**6012 Auburn Avenue**
**Oakland, California 94618(US)**

(54) **Method and reagents for determining the presence of giardia lamblia.**

(57) Methods and reagents are provided for detecting polynucleotide sequences in protozoa causing giardiasis. Primers, probes, compositions and kits for performing the methods, especially by use of a polymerase chain reaction for amplifying a target sequence, are described.

The present invention relates generally to methods and reagents for detecting pathogens capable of causing giardiasis.

The protozoan parasite, *Giardia lamblia (G. duodenalis)* inhabits the small intestine of humans as well as many other mammals. In fact, *Giardia* species parasitize at least 40 species of vertebrates. This parasite is a major cause of human diarrhea in both developed and less developed countries. In the United States, infection occurs among infants and children attending day care centers, travelers, backpackers and campers, and patients that are immunocompromised. In addition, epidemics can occur from contaminated water supplies; from 1986-1988 *G. lamblia* was the major identifiable cause of waterborne disease outbreaks in the United States. Presently, management of the disease is made difficult by insensitive and nonspecific diagnostic tools. Diagnosis of giardiasis relies primarily on microscopic detection of *Giardia* cysts present in fecal specimens. Antibodies reactive with surface antigens are sometimes used for direct fluorescent antibody staining of fecal specimens and in solution based immunoassays. However, cyst excretion is known to be erratic, thus multiple specimens are required for analysis. Duodenal aspirates and biopsies do not provide a greater number of positive results, and are more invasive procedures for the patient. Serodiagnosis by measurement of serum anti-*Giardia* IgG and IgM titers may not discriminate current from previous infections. To further complicate the diagnostic picture, there is a relatively high rate of apparent asymptomatic infection. Additionally, researchers have questioned the criteria for speciation among *Giardia* due to the various mammalian host species, and there have been demonstrations of intrinsic differences among strains of *G. lamblia* that infect humans as well as variation of surface antigens.

Speciation (i.e., the existence of subgroups within the *Giardia* genus) has been the subject of much research and controversy over the years. Traditionally, taxonomic criteria have included cell morphology, cell dimensions, and host specificity. The use of volunteer studies, animal transmission experiments, and cell morphology have supported the concept of a single grouping or species of *Giardia* that is capable of being transmitted to humans and subsequently causing disease therein despite being present and viable in many different mammalian hosts. Recent use of molecular techniques such as electrophoretic karyotyping, isoenzyme analysis, restriction endonuclease analysis, labeling of surface antigens, and epitope mapping with monoclonal antibodies has often identified polymorphism within the generally similar *G. lamblia* grouping. Resolution of these questions has been hampered by the difficulty in obtaining and culturing pure populations of trophozoites and excysting cysts. The polymerase chain reaction (PCR) is particularly applicable to examining specific DNA sequences.

There is thus a clear need for a simple, rapid, sensitive and specific diagnostic technique that can detect *G. lamblia* in fecal and other specimens such as environmental samples (e.g. water, sewage) and clinical specimens (e.g. duodenal aspirates, biopsies, veterinary specimens, etc.). Use of the polymerase chain reaction (PCR) has revolutionized the detection of a wide variety of bacterial, fungal, viral and parasitic pathogens. Enrichment and in vitro culturing of the pathogen is not required, and a relatively crude clinical specimen can provide the source of the nucleic acid for detection and diagnosis. PCR effects the targeted amplificaton of a specific nucleic acid sequence which dramatically increases the number of copies for detection and concomitantly reduces the complexity of the nucleic acid being analyzed. For the detection and diagnosis of pathogenic agents, either a gene encoding for a virulence factor or a universal gene can be targeted for amplification. The small-subunit ribosomal RNA (rRNA) gene is required for viability and contains both conserved and variable gene regions. The selection of primer and probe sequences within the rRNA gene allows for the design of either genus-specific, species-specific or "universal" amplification and detection systems.

The published nucleotide sequences for the *Giardia lamblia* 18S ribosomal RNA gene are set forth in Sogin, M.L., et al., Science 243, 75-777 (1989) (the Sogin reference); and Healey , A., et al., Nucleic Acids Res. 18., 4006 (1990), and a published alignment of prokaryotic and eukaryotic small subunit ribosomal RNA genes (Neefs et al., Nucleic Acids Res. 18, 2237-2317 (1990).

Past attempts to detect *G.lamblia* nucleic acids have employed radiolabeled DNA probes: a genus-specific cloned DNA fragment (Butcher, P.D. and M.J.G. Farthing, Biochem. Soc. Trans. 17, 363-364 (1989)-), a probe mixture of total genomic DNA (Lewis, D.J.M. et al., Lancet 336, 257 (1990)), and most recently a cDNA probe corresponding to a 265 bp fragment of the 18S rRNA gene (Abbaszadegan, M., et al., Appl. Env. Microbiol. 57, 927-931 (1991)). The sensitivity of the first two techniques was 1-10 ng of DNA and $10^4$-$10^5$ cysts or trophozoites. Detection of *G.lamblia* DNA in nucleic acid extracts from fecal specimens was unsuccessful using the total genomic probe. The detection system using the cDNA probe was developed for application to the monitoring of water samples. That assay was capable of detecting 1-5 cysts/ml of water sample concentrates. The cDNA probe test is genus-specific, but had a reduced sensitivity with a bird isolate of *Giardia*, and different levels of sensitivity when applied to human isolates of trophozoites. This is not surprising considering the overall differences between the 18S rRNA sequences of *Giardia* species.

The present invention pertains to methods and reagents for the rapid detection and identification of pathogens causing giardiasis.

In the present invention, an assay utilizing the PCR to detect the presence of *G. lamblia* DNA in fecal specimens was developed and tested using the *G. lamblia* 18S RNA gene sequence as a target. Following optimization of conditions for the PCR, the sensitivity and specificity of the assay was established. A wide variety of isolates obtained from different geographic locations and mammalian hosts were analyzed. Finally, the feasibility of detecting *G.lamblia* in fecal specimens from individuals with parasite infection was evaluated.

The present invention permits the direct detection of *Giardia* trophozoites or cysts in stool specimens and gastric biopsy specimens without the need for microscopic evaluation of specimens and provides a noninvasive method of diagnosis. Current serological tests only measure antibody response to infection, and thus are not good indicators for monitoring therapy or disease reoccurrence.

The present invention also permits the detection of *G. lamblia* in environmental samples such as water and sewage.

In a preferred method, a target region from genomic DNA or complementary DNA transcribed from 18S rRNA is amplified by PCR under defined conditions and using defined primers. The resultant amplified DNA is treated with defined probes. The invention thus also relates to specific probes and their complements for identifying pathogens causing giardiasis. It also pertains to unique oligonucleotide sequences, mutants, fragments and subsequences thereof, from which such specific probes were derived.

In addition, the unique sequences of primers to be used in PCR, as well as the conditions thereof, are described herein.

Further defined herein are nucleotide sequence data for some isolates of *Giardia lamblia* in the region of the 18S rRNA. It was not previously shown that different isolates of this species of *Giardia* contained different sequences within the region of nucleotides 1251-1433. This sequence information was obtained experimentally.

Table 1 shows nucleotide sequence data from the target area 18S rRNA gene segments amplified by primers JW1 (SEQ ID NO:4) and JW2 (SEQ ID NO:5) from *G. lamblia* isolates obtained with DNA from various isolates.

The present invention is a method for determining the presence of and identifying pathogens capable of causing giardiasis by means of hybridizing probes to amplified nucleotide sequences which are characteristic of that pathogen or a species thereof.

The following terms, as used in this disclosure and claims, are defined as:

nucleotide: a subunit of a nucleic acid consisting of a phosphate group, a 5' carbon sugar and a nitrogen containing base. In RNA, the 5' carbon sugar is ribose. In DNA, it is a 2-deoxyribose. The term also includes analogs of such subunits.

nucleotide polymer: at least two nucleotides linked by phosphodiester bonds.

oligonucleotide: a nucleotide polymer generally about 10 to about 100 nucleotide in length, but which may be greater than 100 nucleotides in length.

nucleic acid probe: a single stranded nucleic acid sequence that will combine with a complementary single stranded target nucleic acid sequence to form a double-stranded molecule (hybrid). A nucleic acid probe may be an oligonucleotide or a nucleotide polymer.

hybrid: the complex formed between two single stranded nucleic acid sequences by Watson-Crick base pairings or non-canonical base pairings between the complementary bases.

hybridization: the process by which two complmentary strands of nucleic acids combine to form double stranded molecules (hybrids).

complementarity: a property conferred by the base sequence of a single strand of DNA or RNA which may form a hybrid or double stranded DNA:DNA, RNA:RNA or DNA:RNA through hydrogen bonding between Watson-Crick base pairs on the respective strands. Adenine (A) usually complements thymine (T) or uracil (U), while guanine (G) usually complements cytosine (C).

In accordance with this invention, we have determined the target sequence of *Giardia lamblia* through a comparative analysis of the DNA sequences of various different strains of *G. lamblia*, both published in the literature and sequences we have determined ourselves, utilizing PCR procedures. As a result of our analysis, we have determined the target sequences as illustrated in Table 1. The sequences represent oligonucleotides of from 1251 through 1433, except that the nucleotide of Group 1 (SEQ ID NO:1) at 1277 can be replaced with a G to yield Group 2 [SEQ ID NO:2], and that the nucleotide of Group 1 at 1298 can be replaced by a T to yield Group 3 [SEQ ID NO:3].

In accordance with this invention, we designed oligonucleotide primers sufficiently complementary to a portion of the target sequence as illustrated in Table 1 and its complement thereof, so as to be able to hybridize to a portion of this target sequence as illustrated in Table 1. In accordance with this invention, the primers are designed either for the target sequences illustrated in Table 1 or for their complementary sequences. In this manner, these primers are sufficiently complementary to separate strands of *Giardia lamblia* DNA, so as to hybridize to one of said strands. Each of said primer having the ability to hybridize to a separate DNA strand. In this manner, through the identification of the target nucleic sequence of various strains of *Giardia lamblia* DNA, one can utilize these primers in accordance with the polymerase chain reaction (PCR) to amplify the target nucleic acid sequence of *Giardia lamblia* DNA in fluid samples suspected of containing *Giardia lamblia*. In accordance with this invention, we have found that oligonuclotide primers containing at least 10 nucleotides can be utilized in carrying out this reaction. Generally, these nucleotide primers can contain from about 10 to 30 nucleotides. In accordance with this invention, any primer formed of such oligonuclotides, which is sufficiently complementary to hybridize to a separate strand of the DNA of *Giardia lamblia* to hybridize thereto and when amplified by means of said polymerase chain reaction will form an extension product containing the target DNA sequence. The target DNA sequence can be detected by hybridization to suitable oligonuclotide probes containing at least 14 nucleotides which are substantially complementary to a polynucleotide sequence of said target region. Generally, these oligonuclotide probes contain from about 14 to about 30 nucleotides.

## Table 1

```
                              1251                                                                          1310
G. lamblia  Group 1   GCGCACCAGGAATGTCTTGTAGGCGCCCGCCCCCACCGCGCCGGACGGCGTCCCTGCCC
            Group 2   .........................................G................
            Group 3   ...............................................T..........

                              1311                                                                          1370
G. lamblia  Group 1   CTTGTACACACCGCCCGTCGCTCCTACCGACTGGGCGCCGGCGGACGCCCGGACGCG
            Group 2   ........................................................
            Group 3   ........................................................

                              1371                                                                          1430
G. lamblia  Group 1   CGAAGGGCCGCGAGCCCCCGCGCCTGGAGGAAGGAGAGAAGTCGTAACAAGGTATCCGTAGG
            Group 2   .............................................................
            Group 3   .............................................................

                              1431 TGA  1433
G. lamblia  Group 1   TGA
            Group 2   ...
            Group 3   ...
```

**Group 1:** SEQ ID NO:1. Contained in published sequence for *G. lamblia* (Sogin et al., 1989; Healey et al., 1990). Sequence experimentally determined for WB strain.

**Group 2:** SEQ ID NO:2. Sequence experimentally determined for **Be-1, E-9/M, G1M, PM, CM, GS/M-H7** strains. The isolates with this sequence showed slightly reduced binding to probe RDR34 under stringent wash conditions (described in Example 7).

**Group 3:** SEQ ID NO:3. Sequence experimentally determined for **E-2/M, JH, AB** strains. The isolates with this sequence showed a pronounced diminution of binding to probe RDR34 under stringent wash conditions. Probe RDR317 strongly hybridized to this sequence.

Nucleotide positions in agreement with the Group 1 sequence are designated by dots.

Underlined sequences indicate the location of probes.

The numbers indicate the numbering of the *G. lamblia* 18S rRNA gene.

In accordance with this invention, oligonucleotides containing sequence 1251-1433 as set forth in Groups 1, 2 and 3 of Table 1 (SEQ ID NO:1, SEQ ID NO:2, and SEQ ID NO:3, respectively) were prepared. These oligonucleotides were utilized to design the primers and probes in accordance with this invention.

In accordance with this invention, the test for the presence of *Giardia lamblia* in samples is carried out by treating samples with an aqueous solution containing the oligonucleotide primers which are sufficiently complementary to the separate strands of *Giardia lamblia* DNA to hybridize thereto. In according with this invention, the solution which is utilized to treat the sample could contain as a cosolvent an inert organic polar solvent. Any conventional inert organic polar solvent can be utilized as a component of the aqueous solution. In accordance with the preferred embodiment of this invention the polar solvent, which is used as a cosolvent, can be glycerol, dimethylsulfoxide or formamide, or mixtures thereof. Furthermore, in accordance with the preferred embodiment of this invention, the cosolvent should constitute from about 1% to about 20% by volume of the solution which is utilized to treat the sample to be tested.

The use of specific polynucleotide sequences as probes for the recognition of infectious agents is becoming a valuable alternative to problematic immunological identification assays. For example WO84/02721 describes the use of nucleic acid probes complementary to targeted nucleic acid sequences composed of ribosomal RNA, transfer RNA, or other RNA in hybridization procedures to detect the target nucleic acid sequence. While the assay may provide greater sensitivity and specificity than known DNA hybridization assays, hybridization procedures which require the use of a complementary probe are generally dependent upon the cultivation and/or enrichment of a test organism and are, therefore, unsuitable for rapid diagnosis. Probes can be used directly on clinical specimens if a means of amplifying the DNA or RNA target is available.

The unique sequences of the primers used in the invention will be described herein, as well as preferred or essential PCR conditions.

Polymerase chain reaction (PCR) is a powerful technique that can be used for the detection of small numbers of pathogens whose in vitro cultivation is difficult or lengthy, or as a substitute for other methods which require the presence of living specimens for detection. In its simplest form, PCR is an in vitro method for the enzymatic synthesis of specific DNA sequences, using two oligonucleotide primers that hybridize to opposite strands and flank the region of interest in the target DNA. A repetitive series of cycles involving template denaturation, primer annealing, and the extension of the annealed primers by DNA polymerase results in the exponential accumulation of a specific fragment whose termini are defined by the 5' ends of the primers. PCR reportedly is capable of producing a selective enrichment of a specific DNA sequence by a factor of $10^{12}$. The PCR method is described in Saiki et al., Science 230: 1350 (1985) and is the subject of U.S. Patents Nos. 4,683,195, 4,683,202, 4,800,159 and 4,965,188. This method has been used to detect the presence of the aberrant sequence in the beta-globin gene which is related to sickle cell anemia (Saiki et al. (supra) and human immunodeficiency virus (HIV) RNA (Byrne et al., Nucl. Acids Res 16: 4165 (1988)).

The invention provides methods for determining the presence of a protozoan polynucleotide in a sample suspected of containing polynucleotide, wherein said polynucleotide contains a selected target region, said method comprising:

(a) amplifying the target region, if any, to a detectable level;

(b) providing polynucleotide probes, containing sequences which are substantially complementary to polynucleotide sequences characteristic of species of suspected pathogens in the target region;

(c) incubating the amplified target region, if any, with the polynucleotide probes under conditions which allow specificity of hybrid duplexes; and

(d) detecting hybrids formed between the amplified target region, if any, and the polynucleotide probes.

The methods of the present invention thus enable determination of the presence of the suspected pathogen more rapidly than heretofore possible with prior art detection methods. The basic PCR process is carried out as follows.

A sample is provided which is suspected of containing a particular nucleic acid sequence of interest, the "target sequence". The nucleic acid contained in the sample may be first reverse transcribed into cDNA, if necessary, and then denatured, using any suitable denaturing method, including physical, chemical, or enzymatic means, which are known to those of skill in the art. A preferred physical means for strand separation involves heating the nucleic acid until it is completely (>99%) denatured. Typical heat denaturation involves temperatures ranging from about 80° to about 100°, for times ranging from about 5 seconds to 10 minutes using current technology.

The denatured DNA strands are then incubated with the selected oligonucleotide primers under hybridization conditions, conditions which enable the binding of the primers to the single oligonucleotide strands. As known in the art, the primers are selected so that their relative positions along a duplex sequence are such that an extension product synthesized from one primer, when it is separated from its complement, serves as a template for the extension of the other primer to yield a replicate chain of defined length.

The primer must be sufficiently long to prime the synthesis of extension products in the presence of the agent for polymerization. The exact length of the primers will depend on many factors, including temperature, source of the primer and use of the method. For example, depending on the complexity of the target sequence, the oligonucleotide primer typically contains about 15-30 nucleotides, although it may contain more or fewer nucleotides. Short primer molecules generally require cooler temperatures to form sufficiently stable hybrid complexes with the template. The primers must be sufficiently complementary to hybridize selectively with their respective strands.

The primers used herein are selected to be "substantially" complementary to the different strands of each specific sequence to be amplified. The primers need not reflect the exact sequence of the template, but must be sufficiently complementary to hybridize selectively with their respective strands. Non-complementary bases or longer sequences can be interspersed into the primer, provided that the primer retains sufficient complementarity with the sequence of one of the strands to be amplified to hybridize therewith, and to thereby form a duplex structure which can be extended by the polymerizing means. The non-complementary nucleotide sequences of the primers may include restriction enzyme sites. Appending a restriction enzyme site to the end(s) of the target sequence is particularly helpful for subsequent cloning of the target sequence.

The oligonucleotide primers and probes may be prepared by any suitable method. Methods for preparing oligonucleotides of specific sequence are known in the art, and include, for example, cloning and restriction digestion of appropriate sequences, and direct chemical synthesis. The primers may be labeled, if desired, by incorporating means detectable by spectroscopic, photochemical, biochemical, immunochemical, or chemical means.

Template-dependent extension of the oligonucleotide primer(s) is then catalyzed by a polymerizing agent in the presence of adequate amounts of the four deoxyribonucleoside triphosphates (dATP, dGTP, dCTP, and dTTP) or analogs, in a reaction medium which is comprised of the appropriate salts, metal cations, and pH buffering system. Suitable polymerizing agents are enzymes known to catalyze primer-and template-dependent DNA synthesis. Known DNA polymerases include, for example, E. coli DNA polymerase I or its Klenow fragment, T4 DNA polymerase, *Taq* DNA polymerase, *Tth* DNA polymerase from *Thermus thermophilus* and DNA polymerase from *Thermococcus litoralis*. The reaction conditions for catalyzing DNA synthesis with these DNA polymerases are well known in the art.

The products of the synthesis are duplex molecules consisting of the template strands and the primer extension strands, which include the target sequence. These products, in turn, serve as templates for another round of replication. In the second round of replication, the primer extension strand of the first cycle is annealed with its complementary primer; synthesis yields a "short" product which is bounded on both the 5'-and the 3'-ends by primer sequences or their complements. Repeated cycles of denaturation, primer annealing, and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve.

The PCR method can be performed in a number of temporal sequences. For example, it can be performed step-wise, where after each step new reagents are added, or in a fashion where all of the reagents are added simultaneously, or in a partial step-wise fashion, where fresh reagents are added after a given number of steps.

In a preferred method, the PCR reaction is carried out as an automated process which utilizes a thermostable enzyme. In this process the reaction mixture is cycled through a denaturing step, a primer annealing step, and a synthesis step. A DNA thermal cycler specifically adapted for use with a thermostable enzyme may be employed, which utilizes temperature cycling without a liquid-handling system, thereby eliminating the need to add the enzyme at every cycle. This type of machine is commercially available.

After amplification by PCR, the target polynucleotides may be detected directly by gel analysis provided the target DNA is efficiently amplified and the primers are highly specific to the target region to be amplified. To assure PCR efficiency, glycerol and other solvents such as dimethylsulfoxide (DMSO) and formamide, can be used to increase the sensitivity of the PCR at the amplification level and to overcome problems pertaining to regions of DNA having strong secondary structure. These problems may include (1) low efficiency of the PCR, due to a high frequency of templates that are not fully extended by the polymerizing agent or (2) incomplete denaturation of the duplex DNA at high temperature, due to high GC content. The use of such solvents can increase the sensitivity of the assay at the level of amplification to approximately several picograms of DNA. This level of sensitivity eliminates the need to detect amplified target DNA using a probe, and thereby dispenses with the requirements for labeling of probes, gel electrophoresis, Southern blotting, filter hybridization, washing and autoradiography. The concentration range for glycerol is about 5%-20% (v/v), and the DMSO concentration range is about 3%-10% (v/v).

Alternatively, to attain greater sensitivity the target polynucleotides may be detected by hybridization with a polynucleotide probe which forms a stable hybrid with that of the target sequence under stringent to low stringency hybridization and wash conditions. If it is expected that the probes will be completely

7

complementary (i.e., about 99% or greater) to the target sequence, stringent conditions will be used. If some mismatching is expected, for example if variant strains are expected with the result that the probe will not be completely complementary, the stringency of hybridization may be lessened. However, conditions are chosen which rule out nonspecific/adventitious binding. Conditions which affect hybridization and which select against nonspecific binding are known in the art. Generally, lower salt concentration and higher temperature increase the stringency of binding. For example, it is usually considered that stringent conditions are incubation in solutions which contain approximately 0.1 x SSC, 0.1% SDS, at about 65°C incubation/wash temperature, and moderately stringent conditions are incubation in solutions which contain approximately 1-2 X SSC, 0.1% SDS and about 50°-65°C incubation/wash temperature. Low stringency conditions are 2 X SSC and about 30°-50°C.

An alternate method of hybridization and washing is to perform a low stringency hybridization (5x SSPE, 0.5% SDS) followed by a high stringency wash in the presence of 3M tetramethyl-ammonium chloride (TMACl). The effect of the TMACl is to equalize the relative binding of A-T and G-C base pairs so that the efficiency of hybridization at a given temperature is a function of the length of the polynucleotide. Using TMACl, it is possible to vary the temperature of the wash to achieve the level of stringency desired (see Base composition-independent hybridization in tetramethylammonium chloride: A method for oligonucleotide screening of highly complex gene libraries; Wood, et al., Proc. Natl. Acad. Sci. USA 82, 1585-1588 (1985)).

Probes for target sequences may be derived from the 18S rRNA gene sequences or their complements. The sequence of the 18S *G. lamblia* ribosomal RNA gene has been published by two groups (Sogin, M.L., et al., Science 243, 75-77 (1989); Healey et al., Nucleic Acids Res. 18, 4006 (1990)). The probes may consist of the bases A, G, C or T or analogs (including inosine and 5-methyl-cytosine). The probes may be of any suitable length which span the target region, but which exclude the primers, and which allow specific hybridization to the target region. Generally, the probes will have at least 14 nucleotides, preferably at least 16 nucleotides. The target sequence can come from either of the complementary DNA strands. If there is to be complete complementarity, i.e., if the strain contains a sequence identical to that of the probe, the duplex will be relatively stable under even stringent conditions and the probes may be short, i.e., in the range of about 10-20 basepairs. If some degree of mismatch is expected with the probe, i.e., if it is suspected that the probe will hybridize to a variant region, the probe may be of greater length, since length seems to counterbalance some of the effect of the mismatch(es). The probe may be formed from a subset of the target region and therefore need not span the entire target region. Any subset of the target region has the potential to specifically identify the target region. Further, fragments of the probes may be used so long as they are sufficiently characteristic of the species to be detected. If desired, the probe may also be labeled. A variety of labels which would be appropriate, as well as methods for their inclusion in the probe are known in the art, and include, for example, radioactive atoms, such as $^{32}$P, or other recognizable functionalities e.g., biotin (preferably using a spacer arm), fluorescent dyes, electron-dense reagents, enzymes capable of forming easily detectable reaction products (e.g., alkaline phosphatase, and horseradish peroxidase), or antigens for which specific antisera or monoclonal antibodies are available.

In order to obtain probes to be used for the PCR assays described herein, enough of the nucleotide sequence of the target region must be known. Analysis of the nucleotide sequence of the target region may be direct analysis of the PCR amplified products as described in Gyllensten and Erlich, Proc. Natl. Acad. Sci. USA 85: 7652 (1988) and in U.S. Patent No. 5,066,584; or by cloning the isolated PCR amplified DNA fragments into plasmid vectors and sequencing the DNA obtained from bacteria transformed with insert-containing vector DNA.

It may be desirable to determine the length of the PCR product detected by the probe. This may be particularly true if it is suspected that variant strains may contain deletions or insertions within the target region, or if one wishes to confirm the length of the PCR product. In such circumstances, it is preferable to subject the products to size analysis as well as hybridization with the probe. Methods for determining the size of nucleic acids are known in the art, and include, for example, gel electrophoresis, sedimentation in gradients, and gel exclusion chromatography.

In the present invention, the PCR is used to detect the protozoan parasite, *Giardia lamblia (G. duodenalis)* following *in vitro* amplification of part of the 18S rRNA gene. The system has a high analytical sensitivity; the detection limit using purified nucleic acid is less than one organism's equivalent of DNA. Hybridization with an oligonucleotide probe allows the assay to be specific for the human pathogen, *G. lamblia.* Over thirty different isolates obtained from many geographic locations have been detected by this assay.

The 18S sequences have not been previously used for diagnosis of *Giardia*, nor are there any reports of the use of PCR on this organism. *G. lamblia* represents the earliest diverging lineage in the eukaryotic line of evolution, thus its ribosomal DNA sequences can be readily discerned through the use of PCR from other eukaryotic and prokaryotic organisms. Unexpectedly this PCR system amplified three relatively divergent species of *Giardia*. Use of specific hybridization probes renders the system specific for the *Giardia* species which is pathogenic to humans, *G. lambia*. Unexpectedly, three different sequences were found experimentally among *G. lambia* isolates for the amplified region (see Table 1). This variability provided the sequence basis for sub-species group-specific probes. Additionally, efficient amplification of the target sequence required the presence of cosolvents in the reaction.

The amplification efficiency and thus overall sensitivity of the above-described *Giardia* PCR assay increased several orders of magnitude following optimization of the denaturation temperature, annealing temperature, magnesium concentration, and most importantly the inclusion of two different cosolvents, glycerol and dimethylsufoxide. The use of cosolvents in PCR is believed to enhance amplification of G/C-rich sequences by increasing template denaturation (both initial genomic denaturation and subsequent amplicon denaturation) which affects amount of template available for primer annealing. It also affects the extension of the primers by *Taq* polymerase and facilitates primer extension through secondary structures. For amplification of the G/C-rich *Giardia* target sequence, the principal effect of the cosolvents is believed to be the involvement of template denaturation in primer extension. When DNA sequencing of the cloned 18S amplicons was performed using primers complementary to regions located 20 and 40 bp from the insert within the plasmid vector, the "optimized" conditions were essential for amplification of the cloned insert DNA. In addition, the substitution of the base analog 7-deaza-2'-deoxyguanosine-5'-triphosphate for a portion of the dGTP did not facilitate target amplification, although this base could be incorporated by *Taq* polymerase in the presence of cosolvent, suggesting that the initial cycles involving denaturation of genomic DNA were the limiting steps.

As will be later described, standard amplification conditions resulted in little or no amplification of the target DNA flanked by the primers of the invention. Inclusion of a cosolvent or cosolvents in the PCR amplification mixture has been found necessary to achieve the amplification of the specific DNA target product.

The cosolvent or cosolvents will be added to the reaction in amounts (concentrations) of about 1 to 20% (v/v). Suitable cosolvents include glycerol, dimethylsulfoxide, and formamide, with the former two being preferred. When glycerol and DMSO are used together in an especially preferred embodiment, they will preferably be added in concentrations of of 5-20% (v/v), more preferably 10-15%, most preferably about 10%, for glycerol; and about 3-10%, most preferably about 5%, for DMSO.

In addition, when utilizing these cosolvents in the PCR admixture, optimization of the conditions useful in the present invention will be preferred.

Other preferred modifications of PCR reaction conditions for use herein are as follows: a reduction of the magnesium concentration to 0.75-1.25 mM, preferably 1.0 mM.

The presence of the target sequence in a biological sample is detected by determining whether a hybrid has been formed between the probe and the nucleic acid subjected to the PCR amplification techniques. Methods to detect hybrids formed between a probe and a nucleic acid sequence are known in the art. For example, an unlabeled sample may be transferred to a solid matrix to which it binds, and the bound sample subjected to conditions which allow specific hybridization with a labeled probe; the solid matrix is then examined for the presence of the labeled probe. Alternatively, if the sample is labeled, an unlabeled probe is bound to the matrix, and after exposure to the appropriate hybridization conditions, the matrix is examined for the presence of a label. Saiki et al., Proc Natl Acad Sci, USA 86: 6230-6234 (1989) describe methods of immobilizing multiple probes on a solid support and using hybridization to detect the amplified target polynucleotides of interest. The procedures are well suited to the use of a panel of probes which can provide simultaneous identification of more than one pathogen in a single clinical sample. In another alternative procedure, a solution phase sandwich assay may be used with labeled polynucleotide probes, and the methods for the preparation of such probes are described in U.S. Patent No. 4,820,630.

The probes described herein are preferably applied to the detection of giardiasis by using them to detect and identify what pathogens are present in a sample. The probes described below, as well as additional probes, can be arranged in a reverse dot blot format, as described by Saiki, et al. (supra). Each of the probes is immobilized as a separate dot on a solid support such as a nylon membrane or microtiter plate. The amplified DNA is hybridized to each of the probes at the same time in an aqueous solution. The pattern of the signals from each of the dots (i.e., probes) indicates the identity of the target DNA.

Also within the scope of the present invention are PCR kits for use in carrying out any of the aforementioned PCR processes. The diagnostic kits include the polynucleotide probes and the primers in separate containers. Either of these may or may not be labeled. If unlabeled, the ingredients for labeling may also be included in the kit. The kit may also contain other suitably packaged reagents and material needed for the particular hybridization protocol, for example, standards, and/or polymerizing agents, as well as instructions for conducting the test.

In use, the components of the PCR kit, when applied to a nucleic acid sample, create a reagent mixture which enables the detection and amplification of the target nucleic acid sequence. The reagent mixture thus includes the components of the kit as well as a nucleic acid sample which contains the polynucleotide chain of interest.

A variation of this approach is to use an alternate method of producing the amplified target region. For example, the TAS amplification system, (Kwoh, et al., Transcription-based amplification system and detection of amplified human immunodeficiency virus type I with a bead-based sandwich hybridization format, .Proc. Natl. Acad. Sci. USA 86:1173-1177 (1989)) and its modification, SSSR (Guatelli, et al., Isothermal, in vitro amplification of nucleic acids by a multi-enzyme reaction modeled after retroviral replication, Proc. Natl. Acad. Sci. USA 87: 1874-1878 (1990)) is a method for amplifying RNA or DNA using cycles consisting of a cDNA step to produce a cDNA copy of the template and an RNA transcription step to increase the copy number of the cDNA. This method, like PCR, employs two oligonucleotide primers which hybridize to opposite strands of the target region and flank the target region. The primers described herein may, with minor modifications (the addition of RNA polymerase promoter sequences at the 5' end of one of the primers), be used in a TAS or SSSR amplification system. The subsequent step of the assay, detection by the oligonucleotide probes described herein, may be carried out essentially as described above for the PCR-based assay or may be done using a bead-based sandwich hybridization system.

By way of further specificity, the following probe and primer nucleotide sequence data is provided:

Primer JW1 (SEQ ID NO:4) corresponds to nucleotide base numbers 1251-1270 of the *G. lamblia* 18S ribosomal RNA gene as specified in the Sogin reference.

Primer JW2 (SEQ ID NO:5) corresponds to the complement of nucleotide base numbers 1433-1414 in the *G. lamblia* 18S ribosomal RNA gene as specified in the Sogin reference.

RDR 34 (SEQ ID NO:6), RDR 317 (SEQ ID NO:7) and RDR 35 (SEQ ID NO:8) are oligonucleotide probes for various *Giardia* species. RDR34 (SEQ ID NO:6) and RDR317 (SEQ ID NO:7) correspond to the complement of nucleotide base numbers 1306-1291 of the 18S rRNA gene; RDR35 (SEQ ID NO:8) corresponds to nucleotide base numbers 1363-1378 of the 18S rRNA gene. RDR34 (SEQ ID NO:6) is the primary probe. RDR317 (SEQ ID NO:7) hybridizes to a subset of *G. lamblia* as well as *G. ardeae*. RDR35 (SEQ ID NO:8) is specific for *G.lamblia* but of lower sensitivity.

Table 1 provides a description of nucleotide sequences isolated as described below, which can be used to design and formulate sub-species specific probes and primers corresponding to fragments or subsequences thereof or their complements.

The following examples are intended to be illustrative of the various methods and compounds of the invention.

Example 1

Materials and methods used to obtain DNA for PCR and to obtain sequence data:

A. DNA sources:

DNA was used from the *Giardia* isolates listed in Table 2. Axenic cultures of the isolates were used for extraction of DNA. DNA from *G. lamblia* isolates 3 through 20 listed in Table 2 was obtained from Dr. T. Nash, National Institutes of Health, Bethesda, MD, U.S.A.; DNA from *G. lamblia* isolates 21 through 36, listed in Table 2, *Giardia muris*, *Giardia ardeae*, and *Tritrichomonas fetus* were obtained from Dr. H. van Keulen, Cleveland State University, Cleveland, OH, U.S.A. ATCC strains 30888 (Portland-1 strain) and 30957 (WB strain) (isolates 1 and 2 listed in Table 2) were obtained directly from the ATCC and cultured. DNA was extracted from cells pelleted from those two cultures using sodiumdodecyl sulfate and proteinase K according to the method described in Sambrook, J., et al., Moleculer cloning: a laboratory manual, p. 9.16-9.19(1989), Cold Spring Harbor Laboratory, Cold Spring Harbor, NY, U.S.A.

DNA of *Leishmania major* strain 513 and *Entamoeba histolytica* HM-1 were obtained from Dr. J. Sakanari, University of California at San Francisco, U.S.A.; DNA of *Trypanosoma brucei* 117 and *Toxoplasma gondii* were obtained from Dr. J. Bangs, Stanford University, Stanford, CA, U.S.A.; and *E.coli* DNA and a pool of human placental DNA were obtained from Sigma Co. (St. Louis, MO, U.S.A.).

B. DNA Cloning:

The positive control plasmid, pJW101, containing a 1.02 kB insert of the *G.lamblia* (Portland-1 strain) 18S rRNA gene, was constructed as follows: *Giardia* DNA obtained in Example 1A was amplified from nucleotides 432-1453 by PCR using primers NS3 and NS8 identified by White et al. (1990) Innis et al., ed., PCR protocols, Academic Press, San Diego, CA, U.S.A., and the procedure described in Example 2. The amplified DNA was treated with DNA polymerase I (Klenow fragment). The appropriate size fragment was subsequently gel purified, and blunt-end ligated into *Hinc*II-digested plasmid pUC19 DNA and used to transform competent Dh5αF' cells.

DNA corresponding to nucleotides 1251-1433 of the 18S rRNA gene was amplified by PCR using primers JW1 (SEQ ID NO:4) and JW2 (SEQ ID NO:5) and the procedure described in Example 2 from DNA of ten different G. lamblia isolates (see Table 2 and Example 1). The DNA fragments were isolated and cloned into pUC19 vector DNA as described above.

C. DNA Sequencing:

Plasmid DNA from insert-containing clones was subsequently purified and double-strand sequenced using $c^7$dGTP and a USB Sequenase™ version 2.0 kit. Several clones from each ligation reaction were sequenced, see Table 1 for the DNA sequence results. The *G. lamblia* isolates could be broken into three groups (SEQ ID NO:1, SEQ ID NO:2, and SEQ ID NO:3) on the basis of the DNA sequence of this region of the 18S rRNA gene.

D. Extraction of DNA from formalin-fixed fecal specimens:

Twenty-four formalin-fixed fecal specimens were obtained from patients suspected of having a gastrointestinal parasite infection. The specimens had been analyzed microscopically for parasites. Some of the specimens contained parasites other than or in addition to *G.lamblia* such as *Entamoeba* (*E. coli, E. hartmanni, E. histolytica*), *Ascaris*, *Taenia*, *Trichuris*, *Endoclimaxnana*, *Blastocystis hominis*, *Iodamoeba butchlii*, *Hymenolepsis nana*, *Dientamoeba fragilis*, and *Chilomastix mesnili*. Half of the specimens were microscopically-negative for *G. lamblia*. Total DNA was extracted from the fecal specimens in the following manner using positive displacement pipets to prevent sample to sample contamination: approximately 200 $\mu$l of fecal suspension in 10% buffered formalin was transferred to a 1.5 ml microcentrifuge tube and centrifuged for 5 min. at 15,000xg. The supernatant fluid was removed, leaving an approximately 50 $\mu$l-size pellet. The pellet was resuspended in 50 mM Tris-HCl, pH 8, 10 mM EDTA, 150 mM NaCl, and subjected to a cycle of freezing in a dry ice-ethanol bath and then thawing at 37°C. Subsequently, sodium dodecyl sulfate (SDS) and proteinase K were added to the solution to concentrations of 0.5% and 100 $\mu$g/ml, respectively, and then incubated for 30 min. at 55°C. The specimens were then subjected to an additional two cycles of freezing and thawing, following by heating for 10 min. at 95°C. The mixtures were centrifuged at 750xg to remove the debris, and the supernatant fluid removed and retained. The supernatant fluid was digested with RNase (200 $\mu$g/ml) for 30 min. at 37°C. Subsequently, the DNA in the samples was purified by multiple extractions with phenol:chloroform:isoamyl alcohol (25:24:1) until the interface was clear (3-5 times), and then precipitated with ethanol. The DNA was resuspended in 0.1 ml of 10 mM Tris-HCl, 0.1 mM EDTA, pH 8.0. A 1:100 dilution of the extract was found to be the optimal dilution of DNA to add to the PCR reaction.

Example 2

PCR amplification of *Giardia* DNA:

The sequences of the oligonucleotides used as primers to amplify a 183 bp region of the 18S *G.lamblia* rRNA gene were: JW1, the upstream primer: 5'GCGCACCAGGAATGTCTTGT3' [nucleotides 1251 - 1270] (SEQ ID NO:4) and JW2, the downstream primer: 5'TCACCTACGGATACCTTGTT3' [nucleotides 1433-1414] (SEQ ID NO:5) and were constructed by conventional oligonucleotide synthesis. Amplification was conducted in 100 $\mu$l reactions containing 50 $\mu$l of DNA obtained from the samples in Example 1 and 50 $\mu$l of a 2X mixture containing the other reaction components. The 2x reaction mixture contained 20 mM Tris-HCl, pH 8.3, 100 mM KCl, 2 mM MgCl$_2$, 200 $\mu$g/ml gelatin, 20% (w/v) glycerol, 10% dimethylsulfoxide, a 125 $\mu$M final concentration (each) of dATP, dGTP, dCTP, and dTTP, 2.5U of *Taq* DNA polymerase (Perkin-Elmer Cetus, Norwalk, Conn., U.S.A.), and 25 pmoles of each primer. The reaction mixtures were heated to 95°C for 5 min., followed by 25 cycles of [95°C for 45 sec., 50°C for 30 sec., and 72°C for 2 min.], 10 cycles of [98°C for 45 sec., 50°C for 30 sec., and 72°C for 2 min.], and a final extension time at 72°C for 10 min in a DNA thermal Cycler. The resulting amplification reaction products were analyzed by the procedures of Example 3 for all isolates 1 through 36 of Table 2.

For the DNA extracted from fecal samples, the same procedure as given above was used except that 50 cycles of amplification were employed using the following cycling profile: 95°C for 1 min., 50°C for 1 min., and 72°C for 1 min. The amplified DNA sequences from fecal samples were analyzed by the procedure of Example 3. PCR reactions were set up in a hood in a separate room from where the products of reactions were analyzed. Positive displacement pipets, negative controls in each experiment, and other precautions to prevent contamination were strictly observed in accordance with the suggestions of S. Kwok and R. Higuchi, Nature, 339, 237-238 (1989).

Example 3

Detection and analysis of the amplified product:

Aliquots (10 µl) of the amplification reactions of Example 2 were analyzed by electrophoresis in 3% NuSieve agarose/0.5% Seakem agarose gels, and the DNA was visualized by ethidium bromide staining and illumination with long wave UV light. The size of the specific amplification product was 183bp. Southern hybridization or dot blot analysis with a radiolabled specific oligonucleotide probe was then performed as follows: For Southern hybridization, the ethidium bromide-stained agarose gel was denatured in 0.5 N NaOH/1.5 M NaCl for 15 min. followed by neutralization in 1 M Tris-HCl, pH 7.5/1.5 M NaCl for 15 min. The DNA was transferred to a prewerted nylon membrane (Genatran45) (Plasco, Wobrun, MA, U.S.A.) via capillary transfer overnight using 10XSSPE buffer (1X SSPE is 0.18 M NaCl, 10 mM sodium phosphate, 1 mM EDTA, pH 7.4). For dot blot analysis, 10 µl of the PCR reaction was denatured in 100 µl 0.4N NaOH/25 mM EDTA for 5 minutes at room temperature. 100 µl was spotted onto the nylon membrane using a dot blot apparatus (Bio-Rad, Richmond, CA, U.S.A.) and application of a vacuum. The DNA was crosslinked to the membrane by exposure to ultraviolet light.

Oligonucleotide probes were 5' end labeled with [γ - $^{32}$P]ATP (New England Nuclear, Boston, MA, U.S.A.; specific activity, 6000 Ci/mmol) and T4 polynucleotide kinase and subsequently purified from unincorporated ATP by passage through two successive BioGel P-4 (BioRad, Richmond, CA, U.S.A.) spin columns. The level of radioactivity incorporated into the probe was estimated by measuring Cerenkov radiation in a scintillation counter from 3 µl probe solution without scintillation fluid. The sequences of the probes obtained by elucidating the sequences of Table 1 are RDR34: 5'AGGGACGCGTCCGGCG3' (complement of nucleotides 1291-1306) (SEQ ID NO:6); RDR317: 5'AGGGACGCATCCGGCG3' (complement of nucleotides 1291-1306) (SEQ ID NO:7); RDR35: 5'CGGACGCGCGAAGGGC3' (nucleotides 1362-1377) (SEQ ID NO:8). The filters were prehybridized in a solution containing 5X SSPE, 0.5% SDS 0.5% dextran sulfate for 30 min. at 60°C. A volume of $^{32}$P-labeled probe was added to the hybridization solution to a final concentration of 2X10$^5$ cpm/ml, and incubated for 3 hr. at 60°. Following incubation with the probe, the filters were washed twice at 37°C in 5X SSPE/0.1% SDS for 1 min and 10 min respectively. Subsequently, a stringent wash in a 3M TMACl solution [3M tetramethylammonium chloride, 50 mM Tris-HCl, pH 8.0, 0.1% SDS, 2 mM EDTA] for 10 min. at 51°C was performed. Increased stringency was obtained by substituting a wash temperature of 53°C. TMACl equalizes the relative binding of A-T and G-C base pairs so that the efficiency of hybridization is a function of the probe length. The level of stringency is thus controlled by varying the temperature of the TMACl wash solution. The filters were dried and exposed to X-Omat XAR-5 film with an intensifying screen for 3-18 hours at -70°C. The intensity of the signals obtained with input *Giardia* DNA was compared with that of a negative control reaction of no input template DNA.

Example 4

Effect of cosolvents upon amplification:

Using the published sequences of the 18S rRNA gene of *G.lamblia* (Portland-1 strain) (Sogin et al., Science 243, 75-77 (1989); Healey et al., Nucl. Acids., Res. 18: 4006 (1990)) (SEQ ID NO:1) and an alignment of prokaryotic and eukaryotic small subuinit rRNA genes (Neefs, et al., Nucl. Acids Res. 18, 2237-2317 (1990)-), the oligonucleotide primers were designed to amplify a 183 bp region of the 18S rRNA gene sequence. The primers were carefully selected to specifically avoid the amplification of prokaryotic and human DNA, while residing in moderately conserved regions for protozoan species. Initial PCR experiments employing *G.*

*lamblia* DNA (Portland-1 strain, described in Example 1), 25 pmoles each of primers JW1 (SEQ ID NO:4) and JW2 (SEQ ID NO:5), standard amplification buffer [10 mM Tris-HCl, pH 8.3, 50mM KCl, 1.5 mM MgC12, 0.01% gelatin] and 30 cycles of a cycling profile of 95°C for 45 sec, 50°C for 30 sec, 70°C for 2 min.] preceded by a denaturation step of heating to 95°C for 5 min., and following by a final extension time at 72°C for 10 min., resulted in no amplification as detected by agarose gel electrophoresis and ethidium bromide staining.

Since the target DNA enclosed by primers JW1 (SEQ ID NO:4) and JW2 (SEQ ID NO:5) is G/C-rich (72%) and contains numerous stable hairpin-loop structures, the helix-destabilizing base analog, 7-deaza-2'-deoxyguanosine-5'-triphosphate ($c^7$dGTP) was included in the PCR reaction as a 3:1 $c^7$dGTP:dGTP mixture. *Taq* DNA polymerase incorporates $c^7$dGTP with normal kinetics (Innis et al., Proc. Natl. Acad. Sci. U.S.A. 85: 9436-9440 (1988)) and use of $c^7$dGTP has been observed to facilitate amplification of highly structured sequences (McConlogue et al., Nucl. Acids. Res. 16:9869 (1988)). Therefore, the amplification reaction was performed as described above, with the modification of replacing 75% of the dGTP with $c^7$dGTP. 200 pg, 2 ng, and 20 ng of *G. lamblia* Portland-1 strain DNA were amplified in duplicate reactions for 30 cycles of PCR. Following amplification, the products were detected by agarose gel electrophoresis. No amplification of the desired target sequence was observed. However, when glycerol was included in the amplification reaction described above, at a final concentration of 10%, specific amplification of the *G.lamblia* 183bp product resulted. Ten percent glycerol gave the optimal result (0-15% tested). Inclusion of 5% dimethyl sulfoxide in the PCR reaction mixture additionally increased the yield of specific amplification product. With the inclusion of glycerol, $c^7$dGTP was incorporated into the product during PCR, but with no effect upon yield. The specific amplification product from the 18S rRNA gene is set forth in Table 1.

Example 5

Sensitivity of amplification and probe hybridization:

The detection limit of PCR amplification and probe detection was calibrated following the optimization of reaction conditions, including the addition of two cosolvents, (glycerol and DMSO), an increased denaturation temperature (98°C) in the cycling profile of the last 10 cycles and a magnesium concentration reduced to 1.0mM. Dilutions of genomic *G. lamblia* Portland-1 DNA and of plasmid DNA (pJW101-containing a 1.02 kB segment of the *G.lamblia* 18S rRNA gene) of example 1 in the presence of DNA from $10^6$ *E.coli* cells (2 ng of DNA) were subjected to 35 and 45 cycles of amplification with primers JW1 and JW2 as described in example 2. Increasing the cycle number increased the yield of amplified product obtained from the input *G. lamblia* DNA that was observed by agarose gel electrophoresis and ethidium bromide staining. With more cycles, smaller amounts of input template DNA resulted in observable bands following electrophoresis.

When the amplified DNA was subsequently hybridized following denaturation and application to a nylon membrane, with a $^{32}$P-labeled oligonucleotide probe RDR34 as described in example 3, the limit of detection increased 10-100 fold, as compared to gel electrophoresis. The signal intensity from an 18 hr. film exposure relative to the background signal of a negative control reaction of no imput template DNA constituted the limit of detection. The limit of detection following amplification and hybridization was approximately 10-100 copies of target DNA, which is equivalent to ≦1 trophozoite's content of genomic DNA. A trophozoite contains approximately 63 copies of the 18S rRNA gene.

Example 6

Specificity of Amplification and probe hybridization:

The specificity of the PCR amplification was evaluated by testing 2 ng of DNA obtained from five protozoan parasites [*Entamoeba histolytica*, *Leishmania major*, *Trypanosoma brucei*, *Toxoplasma gondii*, *Tritrichomonas fetus*], 2 ng of *E. coli* DNA, 25 ng of human placental DNA, DNA extracted from a normal (control) fecal specimen, and 200 fg-2 ng of the *G.lamblia* Portland-1 DNA described in Example 5. All samples of DNA obtained as described in Example 1, were subjected to amplification by PCR with primers JW1 (SEQ ID NO:4) and JW2 (SEQ ID NO:5) in accordance to the procedures of Example 2. When the amplification reactions were analyzed by gel electrophoresis as described in Example 3, no amplification of the human, *E. coli* or DNA extracted from the fecal specimen was observed. A specific amplification product was obtained from the *E.histolytica*, *L.major*, and *T. fetus* DNA, although the size of the products was larger than that of *G.lamblia*. *G.lamblia* contains the smallest 18S rRNA gene sequenced to date.

The amplified DNA was then subjected to Southern hybridization anaylsis with probe RDR34 (SEQ ID NO:6) by the procedure described in Example 3 to assess the specificity of probe binding. No binding to any other DNA was observed, demonstrating that probe RDR34 (SEQ ID NO:6) is specific for the *Giardia* genus. In addition, DNA isolated from two other species of *Giardia* (*G. muris* and *G. ardeae*) as described in Example 1 was also analyzed using the above procedures. Primers JW1 (SEQ ID NO:4) and JW2 (SEQ ID NO:5) amplified a specific product from both *G. muris* (obtained from the hamster) and *G. ardeae* (obtained from the great blue heron), as detected by gel electrophoresis and the production of a single product of approximately 185-200 bp in size. Southern hybridization anaylsis with probe RDR34 (SEQ ID NO:6) as described above, demonstrated that the probe did not hybridize to the amplified products from these two *Giardia* species. Thus, RDR34 (SEQ ID NO:6) specifically hybridizes only to the *Giardia* species that infects human hosts, *Giardia lamblia*.

Example 7

Amplification and detection of other *G.lamblia* isolates:

DNA obtained from 32 additional isolates of *G. lamblia* (*G. duodenalis*) (in addition to the Portland-1 and WB strains) all set forth in Example 1 were similarly evaluated by PCR and hybridization with probe RDR34 (SEQ ID NO:6) using the procedures described in Examples 2 and 3. All of the *G. lamblia* DNAs produced the specific amplification product identified by gel electrophoresis. The oligonucleotide probe RDR34 (SEQ ID NO:6) specifically hybridized to all of the amplified products from the various *G. lamblia* isolates. Unexpectedly, after very stringent wash conditions were employed in a Southern hybridization analysis (53°C TMACl wash temperature), three different subsets of *G. lamblia* were delineated on the basis of the signal intensity remaining from bound probe RDR34 (SEQ ID NO:6); the results are shown in Table 2.

Subsequently, the nucleotide sequence differences between the three groups of G. lamblia were demonstrated. The DNA sequence of the amplified fragments from ten different isolates that contained members of each subgroup (indicated by asterisks in Table 2) was obtained following cloning into pUC19 DNA, according to the procedure described in Example 1. The results of DNA sequencing are presented in Table 1 and are classified into Group 1 (SEQ ID NO:1), Group 2 (SEQ ID NO:2) and Group 3 (SEQ ID NO:3). As seen in Table 1, each group of DNA contained a single base pair change relative to the other groups.

Probe RDR317 (SEQ ID NO:7) was designed to preferentially hybridize to the Group 3 DNAs, as a substitution for probe RDR34 (SEQ ID NO:6). When all of the above amplified DNAs were subjected to hybridization with probe RDR317 (SEQ ID NO:7) under very stringent conditions according to the procedures described above, the probe only hybridized to the DNA from isolates in Group 3 (SEQ ID NO:3), as expected. The results are set forth in Table 2.

EP 0 550 883 A2

**Table 2.** Giardia isolates

| Isolate | Geographic Origin | Host | Probe RDR34 | Hybridization RDR317 |
|---|---|---|---|---|
| 1. Portland-1 (ATCC 30888) | U.S.A. | human | + | - |
| 2. WB (ATCC 30957) | Afghanistan | human | + | - |
| 3. Be-1 (IP-0482:1)* | Canada | beaver | +[1] | - |
| 4. Be-2 (IP-0583:1) | Canada | beaver[b] | + | - |
| 5. E-4/M (NIH0285:4) | Egypt | human | + | - |
| 6. E-2/M (NIH0285:2)* | Egypt | human | +[2] | + |
| 7. E-9/M (NIH0285:9)* | Egypt | human | +[1] | - |
| 8. G1M* | Peru | human | +[1] | - |
| 9. G2M | Peru | human | + | - |
| 10. G3M | Peru | human | + | - |
| 11. PM (NIH0988)* | U.S.hA. | human | +[1] | - |
| 12. WB (NIH1179)* | Afghanistan | human | + | - |
| 13. Isr (NIH1183) | U.S.A. | human | + | - |
| 14. CAT-1 | U.S.A. | cat | + | - |
| 15. GP | U.S.A. | guinea pig | + | - |
| 16. CM (NIH0883:2)* | U.S.A. | human | +[1] | - |
| 17. JH (NIH1182)* | U.S.A. | human | +[2] | + |
| 18. N (NIH0782) | U.S.A. | human | +[2] | + |
| 19. AB (NIH0883:1)* | Peru | human | +[2] | + |
| 20. GS/M-H7 (NIH1083:2)* | U.S.A. | human | +[1] | - |
| 21. Portland-1 CCW | U.S.A. | human | + | - |
| 22. Portland-1 | U.S.A. | human | + | - |
| 23. BG | Canada | beaver | + | - |
| 24. H-1-P | U.S.A. | human | + | - |
| 25. H-2-P | U.S.A. | human | + | - |
| 26. WB | Afghanistan | human | + | - |
| 27. B5 | Canada | beaver | + | - |
| 28. MR4 | Canada | muskrat[b] | + | - |

Table 2. Giardia isolates (con't)

| Isolate | Geographic Origin | Host | Probe RDR34 | Hybridization RDR317 |
|---|---|---|---|---|
| 29. D3 | Canada | dog | + | - |
| 30. P1/C | Canada | human | + | - |
| 31. CAM | Cambodia | human | + | - |
| 32. S1 | Canada | sheep | + | - |
| 33. LCP/New Orleans-1c | U.S.A. | human | + | + |
| 34. CDC | U.S.A. | human | + | - |
| 35. B5 | Canada | beaver | + | - |
| 36. CH3 | Canada | human | + | - |
| 37. G. muris | U.S.A. | hamster | - | - |
| 38. G. ardeae | U.S.A. | blue heron | - | + |

* Isolates used for purification of amplified DNA fragment and sequence analysis (see Table 1).

a Some isolate names have multiple listings in this table, however these strains were propagated in independent laboratories and may have acquired different properties during extensive in vitro culture. Detailed information concerning the origin of isolates has been published [Isolate nos. 2-20 (Nash, T.E., et al., Mol. Biochem. Parasitol 42, 125-132 (1990)); isolate nos. 21-36 (Van Keulen, H., et al., Mol. Biochem. Parasitol. 46, 275-284 (1991)); isolate no. 38 (Erlandsen, S.L., et al., J. Parasitol. 76, 717-724 (1990))].

b putative, may be human

c Possible mixture of ATCC 50137 and ATCC 50184

+[1] slightly reduced hybridization under very stringent wash conditions

+[2] pronounced dimunition of hybridization under very stringent wash conditions

- no binding of probe under stringent wash conditions

EP 0 550 883 A2

Example 8

PCR assay of fecal specimens:

Formalin-fixed fecal specimens obtained from patients suspected of having an infection with a gastrointestinal parasite were assayed by PCR with primers JW1 (SEQ ID NO:4) and JW2 (SEQ ID NO:5) (in accordance with the procedures of Examples 2 and 3) for the presence of *G.lamblia* DNA. Half of the specimens were microscopically positive for *G.lamblia* (ranging in amount of trophozoites and cysts), while the other twelve were microscopically-negative for *Giardia* but often contained other parasites. Total DNA was extracted from the fecal specimens using a standard technique for extracting DNA from bacterial cells [Silhavy, T.J., et al, Experiments with gene fusions, p. 137-139 (1984), . Cold Spring Harbor Laboratory. Cold Spring Harbor, NY, U.S.A.] in combination with several freeze-thaw cycles to lyse cysts, according to the procedure of Example 1. An aliquot of the isolated DNA was subjected to PCR followed by transfer to a nylon membrane and hybridization with $^{32}$P-oligonucleotide probe (RDR34, RDR35, RDR317) in accordance with the procedures of Examples 2 and 3. Eight of the 12 microscopically-positive specimens were consistently positive following PCR and hybridization with probes RDR34 (SEQ ID NO:6) and RDR35 (SEQ ID NO:8), while ten out of the 12 microscopically-negative specimens were consistently negative. The other four microscopically-positive specimens were not consistently detected as positive, and two of the microscopically-negative specimens were positive by the above analyses.

The inconsistent results with the four microscopically-positive samples could be the result of difficulties in obtaining independent equal samples from a fecal specimen containing only low levels of *Giardia*. The two potentially false-positive samples could be due to the high sensitivity of PCR as compared with microscopic diagnosis techniques. Negative control reactions were alway negative in every experiment indicating that the above were not false-positives due to contamination.

Using purified Portland-1 strain *G. lamblia* DNA as template DNA for PCR, hybridization with probe RDR35 (SEQ ID NO:8) resulted in a weaker signal as compared with RDR34 (SEQ ID NO:6). When the DNA from the fecal samples was analyzed by PCR and probe hybridization, the signal obtained with RDR35 (SEQ ID NO:8) was not always less than that of RDR34 (SEQ ID NO:6). Amplified DNA from those particular samples hybridized with probe RDR317 (SEQ ID NO:7) indicating the presence of Group 3 *G. lamblia* DNA (SEQ ID NO:3) in those fecal specimens.

SEQUENCE LISTING

- INFORMATION FOR SEQ ID NO:1:

    (i) SEQUENCE CHARACTERISTICS:
        (A) LENGTH: 183 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iv) ANTI-SENSE: NO

    (vi) ORIGINAL SOURCE:
        (A) ORGANISM: Giardia lamblia

    (x) PUBLICATION INFORMATION:
        (A) AUTHORS: Sogin, M. L.
        (C) JOURNAL: Science
        (D) VOLUME: 243
        (G) DATE: 1989

    (xi) SEQUENCE DESCRIPTION: SEQ ID NO:1:

```
GCGCACCAGG AATGTCTTGT AGGCGCCCGC CCCCACCGCG CGCCGGACGC GTCCCTGCCC        60

CTTGTACACA CCGCCCGTCG CTCCTACCGA CTGGGCGCGG CGGCGAGCGC CCCGGACGCG       120

CGAAGGGCCG CGAGCCCCCG CGCCTGGAGG AAGGAGAAGT CGTAACAAGG TATCCGTAGG       180

TGA                                                                    183
```

- INFORMATION FOR SEQ ID NO:2:

   (i) SEQUENCE CHARACTERISTICS:
      (A) LENGTH: 183 base pairs
      (B) TYPE: nucleic acid
      (C) STRANDEDNESS: single
      (D) TOPOLOGY: linear

   (ii) MOLECULE TYPE: cDNA

   (iii) HYPOTHETICAL: NO

   (iv) ANTI-SENSE: NO

   (vi) ORIGINAL SOURCE:
      (A) ORGANISM: Giardia lamblia

   (xi) SEQUENCE DESCRIPTION: SEQ ID NO:2:

```
GCGCACCAGG AATGTCTTGT AGGCGCGCGC CCCCACCGCG CGCCGGACGC GTCCCTGCCC    60

CTTGTACACA CCGCCCGTCG CTCCTACCGA CTGGGCGCGG CGGCGAGCGC CCCGGACGCG   120

CGAAGGGCCG CGAGCCCCCG CGCCTGGAGG AAGGAGAAGT CGTAACAAGG TATCCGTAGG   180

TGA                                                                183
```

- INFORMATION FOR SEQ ID NO:3:

   (i) SEQUENCE CHARACTERISTICS:
      (A) LENGTH: 183 base pairs
      (B) TYPE: nucleic acid
      (C) STRANDEDNESS: single
      (D) TOPOLOGY: linear

   (ii) MOLECULE TYPE: cDNA

   (iii) HYPOTHETICAL: NO

   (iv) ANTI-SENSE: NO

   (vi) ORIGINAL SOURCE:
      (A) ORGANISM: Giardia lamblia

   (xi) SEQUENCE DESCRIPTION: SEQ ID NO:3:

```
GCGCACCAGG AATGTCTTGT AGGCGCCCGC CCCCACCGCG CGCCGGATGC GTCCCTGCCC    60

CTTGTACACA CCGCCCGTCG CTCCTACCGA CTGGGCGCGG CGGCGAGCGC CCCGGACGCG   120

CGAAGGGCCG CGAGCCCCCG CGCCTGGAGG AAGGAGAAGT CGTAACAAGG TATCCGTAGG   180

TGA                                                                183
```

- INFORMATION FOR SEQ ID NO:4:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iv) ANTI-SENSE: NO

    (xi) SEQUENCE DESCRIPTION: SEQ ID NO:4:

GCGCACCAGG AATGTCTTGT         20

- INFORMATION FOR SEQ ID NO:5:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iv) ANTI-SENSE: YES

    (xi) SEQUENCE DESCRIPTION: SEQ ID NO:5:

TCACCTACGG ATACCTTGTT         20

- INFORMATION FOR SEQ ID NO:6:

    (i) SEQUENCE CHARACTERISTICS:
        (A) LENGTH: 16 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iv) ANTI-SENSE: YES

    (xi) SEQUENCE DESCRIPTION: SEQ ID NO:6:

AGGGACGCGT CCGGCG         16

- INFORMATION FOR SEQ ID NO:7:

    (i) SEQUENCE CHARACTERISTICS:
        (A) LENGTH: 16 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iv) ANTI-SENSE: YES

    (xi) SEQUENCE DESCRIPTION: SEQ ID NO:7:

AGGGACGCAT CCGGCG                  16

(2) INFORMATION FOR SEQ ID NO:8:

    (i) SEQUENCE CHARACTERISTICS:
        (A) LENGTH: 16 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iv) ANTI-SENSE: NO

    (xi) SEQUENCE DESCRIPTION: SEQ ID NO:8:

CGGACGCGCG AAGGGC                  16

## Claims

1. Method for determining the presence of Giardia lamblia in a sample suspected of containing Giardia lamblia through the use of a target nucleic acid sequence of Giardia lamblia DNA, comprising:
   (a) amplifying a target sequence containing a sequence within the region of nucleotides 1251-1433 of the 18S rRNA gene to a detectable level and
   (b) detecting the amplified product.

2. Method for determining the presence of Giardia lamblia in a sample suspected of containing Giardia lamblia through the use of a target nucleic acid sequence of Giardia lamblia DNA, comprising:
   (a) amplifying a target sequence containing a sequence within the region of nucleotides 1251-1433 of the 18S rRNA gene to a detectable level;
   (b) providing oligonucleotide probes containing sequences which are substantially complementary to oligonucleotide sequences within the target sequence;
   (c) incubating the amplified target sequence with the oligonucleotide probes under conditions which cause hybridization between the probes and the target sequence; and
   (d) detecting hybrids, if any, formed between the amplified target sequence and the oligonucleotide probe.

3. Method of claim 1 or 2, wherein step (a) is performed using a PCR technique.

4. Method of claim 3, wherein one of the primers used in PCR contains from 10-30 nucleotides and comprises at least a part of the sequence of SEQ ID No: 4 and the other of the primers used in PCR contains from 10-30 nucleotides and comprises at least a part of the sequence of SEQ ID No: 5.

5. Method of claim 4 wherein one of said primers contains the sequence of SEQ ID No: 4 and the other of said primers contains the sequence of SEQ ID No: 5.

6. Method of claim 2 wherein the probe contains from 14-30 nucleotides comprising at least a part of the complement of the nucleotides 1251-1433 of the 18S rRNA gene.

7. Method of claim 6, wherein the probe contains the sequence of SEQ ID No: 6, SEQ ID No: 7 or SEQ ID No: 8.

8. Oligonucleotide primer capable of hybridizing to one of the two complementary strands of Giardia lamblia DNA which contains a target sequence within the region of nucleotides 1251-1433 of the 18S rRNA gene.

9. Primer of claim 8 which contains from 10-30 nucleotides and comprises at least a part of the sequence of SEQ ID No: 4 or SEQ ID No: 5.

10. Primer of claim 9 which contains the sequence of SEQ ID No: 4 or SEQ ID No: 5.

11. Oligonucleotide probe capable of hybridizing to one of the two complementary strands of Giardia lamblia DNA which contains a target sequence within the region of nucleotides 1251-1433 of the 18S rRNA gene.

12. Probe of claim 11, wherein said probe contains at least 14 nucleotides and is at least 75% complementary to said target sequence.

13. Probe of claim 12 wherein said probe contains at least a part of the oligonucleotide of SEQ ID No: 6 or its complement.

14. Probe of claim 12 wherein said probe contains at least a part of the oligonucleotide of SEQ ID No: 7 or its complement

15. Probe of claim 12 wherein said probe contains at least a part of the oligonucleotide of SEQ ID No: 8 or its complement.

16. Composition consisting of an aqueous solution containing a pair of oligonucleotide primers, wherein said primers are capable of hybridizing to the different complementary strands of a target nucleic acid sequence of Giardia lamblia DNA which target sequence contains a sequence within the region of nucleotides 1251 - 1433 of the 18S rRNA gene, whereby said primers through hybridization with said strands and subsequent extension act as a template for amplifying said target sequence.

17. Composition of claim 16, wherein the solution contains as a cosolvent an inert organic polar solvent.

18. Composition of claim 17, wherein the solution contains from about 1% to 20% by volume of said cosolvent.

19. Composition of claim 17 or 18, wherein the cosolvent is glycerol, dimethylsulfoxide or formamide.

20. Composition of any one of the claims 16 to 19 wherein one of said primers contains from 10-30 nucleotides and comprises at least a part of the sequence of SEQ ID No: 4 and the other of the primers contains from 10-30 nucleotides and comprises at least a part of the sequence of SEQ ID No: 5.

21. Composition of claim 20 wherein one of said primers contains the sequence of SEQ ID No: 4 and the other of said primers contains the sequence of SEQ ID No: 5.

22. Method of claim 3 wherein in step (a) the composition of any one of claims 16-21 is used.

23. Kit comprising at least one primer as defined in any one of claims 8-10.

24. Kit of claim 23 further comprising a probe as defined in any one of claims 11-15.

25. Kit comprising a composition of any one of claims 16-21.

26. Target nucleic acid sequence for determining the presence of Giardia lamblia in a sample wherein the sequence corresponds essentially or exactly to a sequence selected from the group consisting of SEQ ID No: 1, SEQ ID No: 2 and SEQ ID No: 3 or the complement thereof.

27. The invention as hereinbefore described.